# EUROPEAN PATENT APPLICATION

(11) **EP 4 068 408 A1**
(43) Date of publication of application: **05.10.2022**
(21) Application number: 21894818.0
(22) Date of filing: 06.08.2021
(51) Int. Cl.: H01L 51/52, H01L 51/50, A61N 5/06

(54) **TANDEM BIO-ORGANIC LIGHT-EMITTING DIODE FOR PHOTODYNAMIC THERAPY AND PHOTODYNAMIC APPARATUS COMPRISING SAME**

(30) Priority: 20.11.2020 KR 20200157003
(71) Applicant: DSLAB, Inc., Gyeonggi-do 16419 (KR); Research & Business Foundation Sungkyunkwan University, Gyeonggi-do 16419 (KR)
(72) Inventor: JO, Deok Su, Suwon-si Gyeonggi-do 16417 (KR); CHUNG, Ho Kyoon, Yongin-si Gyeonggi-do 17169 (KR); CHO, Sung Min, Gunpo-si Gyeonggi-do 15817 (KR); KIM, Min Jung, Suwon-si Gyeonggi-do 16419 (KR); DANG, Thi My Linh, Suwon-si Gyeonggi-do 16362 (KR); TRAN, Thien Tri, Suwon-si Gyeonggi-do 16417 (KR)
(74) Representative: Gottschald Patentanwälte Partnerschaft mbB
(86) International application number: PCT/KR2021/010426
(87) International publication number: WO 2022/108041

(57) **Abstract**

The present invention is directed to a tandem-structured bio-organic light emitting diode (bio-OLED) for photodynamic therapy, and to a photodynamic therapy device including the same to convert a red light emitted from the bio-organic light emitting diode into a near-infrared light (NIR) emission, thereby enabling the use of a PBM therapy in a wide wavelength range of 600 to 1,000 nm.

## Description

### TECHNICAL FIELD

The present invention relates to a tandem-structured bio-organic light emitting diode ("bio-OLED") for photodynamic therapy and a photodynamic therapy device including the same.

### BACKGROUND ART

Currently, in the medical field, phototherapy is attracting attention as a means of health improvement or treatment. Phototherapy is a technique that activates, regenerates, or destroys specific tissues in the skin by absorbing light into the skin of the human body. As a method for phototherapy, photobiomodulation (PBM) uses an infrared region or a near-infrared NIR region in wavelength ranges of 600 to 700 nm and 780 to 1100 nm, respectively, and a low-power light source (e.g., a laser or a light emitting diode ("LED")) with an output power in a range of 1 to 500 mW.

Such a light emitting diode (LED) with high optical power generates heat due to phonon (e.g., crystal lattice) vibration, and when the skin is applied with the heat or is exposed to a protein modification temperature (42°C) or higher, side effects such as burns and skin tissue deformation may occur. In addition, a method for lowering the temperature by reducing the optical power of the LED has a disadvantage in that the light for the photodynamic therapy is not sufficiently provided and thus the therapeutic effect is insufficient.

### Prior art literature

Korean Patent Registration KR 10-1660388 B1

### DETAILED DESCRIPTION OF THE INVENTION

### TECHNICAL OBJECTIVES

In a PBM therapy, cytochrome c oxidase ("CCO"), an enzyme that absorbs light from cells in the body, changes its spectrum depending on oxidation and reduction states, but it is distributed in red light and near-infrared ("NIR") where the red light exhibits the spectrum with a center at 600 nm and 660 nm and the NIR exhibits the spectrum with a center at 825 nm. The spectra of 600 nm, 660 nm, and 850 nm are represented by full width at half maximum ("FWHM") of 30 nm, 65 nm, and 175 nm, respectively. However, in the PBM therapy, light at 580 nm or less is absorbed by haemoglobin and oxyhaemoglobin, which may interfere with light absorption in CCO and may cause side effects due to heating of blood. Irradiation of light absorbed by CCO contributes significantly to the generation of adenosine triphosphate ("ATP") and reactive oxygen species ("ROS"). In addition, ATP is a precursor of DNA and RNA, and is also used as a coenzyme.

Accordingly, the present inventors adjusted thicknesses of a hole injection layer (HIL) and a hole transport layer (HTL) constituting an OLED to control a half width of an emission wavelength for the photodynamic therapy, thereby adjusting a peak wavelength of a light emitted from the OLED from 600 nm to 660 nm in terms of full width at half maximum ("FWHM"), and configured the OLED in a tandem structure, thereby completing a red OLED that may emit a wider full width at half maximum.

The present inventors also converted a red light emitted from the red OLED into a near-infrared NIR light emission by using a photo-conversion material, thereby completing a photodynamic therapy device that may use a PBM therapy in a wide wavelength range from 600 nm to 1,000 nm.

The present inventors also completed a light conversion film having excellent light conversion efficiency from a red light to a near-infrared NIR light emission as it is manufactured by an adhesive transfer ("AT") method for electrospinning a fluorescent material that emits IR in the form of a solution.

### TECHNICAL SOLUTION TO THE PROBLEM

The present invention is directed to a tandem-structured bio-organic light emitting diode ("bio-OLED") for photodynamic therapy.

The present invention is also directed to a photodynamic therapy device including the tandem-structured bio-OLED for photodynamic therapy.

The present invention is also directed to a tandem-structured bio-OLED array for photodynamic therapy.

The present invention is also directed to a method for manufacturing a light conversion film having excellent light conversion efficiency into near-infrared NIR.

### EFFECTS OF THE INVENTION

The tandem-structured bio-organic light emitting diode ("bio-OLED") device for photodynamic therapy according to the present invention may form an organic light emitting diode ("OLED") unit layer for emitting a red light (RED) in a tandem structure, thereby having excellent efficiency with improved voltage, luminance, and optical power density, and may also increase an overlap area with cytochrome c oxidase ("CCO") to be applicable to the photodynamic therapy with excellent enzymatic compatibility. In addition, a photodynamic therapy device including the tandem-structured bio-OLED according to the present invention may include a light conversion layer that converts the red light (RED) emitted from the bio-OLED into a near-infrared NIR emission, thereby enabling the use of a PBM therapy in a wide wavelength range from 600 nm to 1,000 nm.

### BRIEF DESCRIPTION OF THE DRAWINGS

FIG. 1 illustrates an exemplary embodiment of a photodynamic therapy device including a tandem-structured bio-OLED ("bio-OLED") according to the present invention.
FIG. 2 illustrates an exemplary embodiment of an NDP layer and an RDP layer of the bio-OLED for photodynamic therapy according to the present invention.
FIG. 3 illustrates an exemplary embodiment of a tandem-structured bio-OLED array for photodynamic therapy according to the present invention.
FIG. 4 illustrates a red OLED structure prepared according to Embodiment 1 of the present invention.
FIG. 5 illustrates voltage, luminance, and optical power density of a red OLED structure prepared according to Embodiment 1 of the present invention.
FIG. 6 illustrates spectral wavelengths of red OLED structures 1 and 3 prepared according to Embodiment 1 of the present invention.
FIG. 7 illustrates spectral wavelengths of red OLED structures 2 and 4 prepared according to Embodiment 1 of the present invention.
FIG. 8 illustrates FE-SEM images of quantum dot beads and fibers prepared according to Embodiment 3 of the present invention.
FIG. 9 illustrates an optical microscope image of a light conversion film prepared according to Embodiment 4 of the present invention.
FIG. 10 illustrates a spectral wavelength of a red OLED to which a light conversion film prepared according to Experimental Example 1 of the present invention is attached.
FIG. 11 illustrates a spectral wavelength of a red OLED to which a light conversion film prepared according to Experimental Example 2 of the present invention is attached.
FIG. 12 illustrates a spectral wavelength of a red OLED to which a light conversion film prepared according to Experimental Example 3 of the present invention is attached.

### PREFERRED MODES FOR IMPLEMENTATION OF THE INVENTION

Hereinafter, a tandem-structured bio-organic light emitting diode ("bio-OLED") for photodynamic therapy and a photodynamic therapy device including the tandem-structured bio-OLED according to specific embodiments of the present invention will be described in detail. This, however, is presented as an example of the invention, thereby not limiting the scope of the invention, and it is apparent to those skilled in the art that various modifications to the embodiment are possible within the scope of the invention.

Throughout this specification, unless otherwise specified, "including" or "containing" refers to including an element (or component) without particular limitation, and it may not be construed as excluding addition of another element (or component).

According to a first embodiment,
the present invention is directed to a tandem-structured bio-OLED for photodynamic therapy, the bio-OLED including:
a first electrode layer;
a second electrode layer formed to face the first electrode;
an organic light emitting diode ("OLED") unit layer disposed between the first electrode layer and the second electrode layer and emitting a red light (RED); and
a charge generation layer ("CGL") disposed between each OLED unit layer.

In the tandem-structured bio-OLED according to the present invention, the OLED unit layer includes at least two or more OLED unit layers formed between the first electrode layer and the second electrode layer.

In the tandem-structured bio-OLED according to the present invention, the OLED unit layer emits a broadband emission spectrum with a full width at half maximum ("FWHM") of about 80 nm or more, preferably 90 nm or more, and more preferably 100 nm or more.

In the tandem-structured bio-OLED according to the present invention, the first electrode layer is transparent or translucent (e.g., semi-transparent), has a thickness in a range of 10 to 100 nm, and includes or is made of Mg:Ag, Al, Cu, ITO, IZO, Mg or Ca.

In the tandem-structured bio-OLED according to the present invention, the second electrode layer has a thickness in a range of 10 to 200 nm and includes or is formed of Mg:Ag, Al, Cu, Mg or Ca.

In the tandem-structured bio-OLED according to the present invention, the charge generation layer is formed of aluminum (Al) having a thickness in a range of 1 to 2 nm.

In the tandem-structured bio-OLED according to the present invention, the OLED unit layer includes at least one of: a hole injection layer (HIL), a hole transport layer (HTL), an emission layer (EML), a hole block layer (HBL), an electron transport layer (ETL) and an electron injection layer (EIL). For example, the OLED unit layer may include a hole injection layer (HIL) formed on the first electrode layer, a hole transport layer (HTL) formed on the hole injection layer, an emission layer (EML) formed on the hole transport layer and emitting a red light (RED), a hole block layer (HBL) formed on the emission layer, an electron transport layer (ETL) formed on the hole block layer, and an electron injection layer (EIL) formed on the electron transport layer.

In the tandem-structured bio-OLED according to an embodiment of the present invention, the hole injection layer may include or be formed of hexacyanohexaazatriphenylene (HAT-CN) having a thickness in a range of 1 to 150 nm. In the tandem-structured bio-OLED according to the present invention, the hole transport layer may include or be formed of NPB (N,N'-diphenyl-N,N'-bis(1,1'-biphenyl)-4,4'-diamine), TCTA, TAPC or mCP with a thickness in a range of 1 to 50 nm. The emission layer may have a thickness in a range of 30 to 40 nm and may emit a red light having a wavelength in a range of 600 to 700 nm. The emission layer may include or be formed of one of: Ir(piq)₃, Ir(tiq)₃, Ir(fliq)₃, Ir(btpy)₃, and Ir(t-5t-py)₃. The hole block layer may include or be formed of BAlq₂ (bis(2-methyl-8-quinolinolate)-4-(phenylphenolato)aluminium) having a thickness in a range of 1 to 10 nm. The electron transport layer may include or be formed of Alq₃ (tris(8-hydroxyquinoline) aluminum) having a thickness in a range of 10 to 30 nm. The electron injection layer may include or be formed of lithium quinolate (Liq) having a thickness in a range of 1 to 3 nm.

According to a second embodiment,
the present invention is directed to a photodynamic therapy device including a tandem-structured bio-OLED, the photodynamic therapy device including:
(A) an encapsulation substrate layer,
(B) a tandem-structured bio-OLED layer including a first electrode layer on the encapsulation substrate layer; a second electrode layer formed to face the first electrode layer; an OLED unit layer disposed between the first electrode layer and the second electrode layer and emitting a red light (RED); and a charge generation layer CGL disposed between each OLED unit layer,
(C) a transparent encapsulation substrate layer formed on the bio-OLED layer, and
(D) a light conversion layer on, or on and below the transparent encapsulation substrate layer,
where the light conversion layer converts the red light emitted from the bio-OLED layer into a near-infrared NIR light emission.

In the photodynamic therapy device including the tandem-structured bio-OLED according to the present invention, the photodynamic therapy device emits a red light and a near-infrared light NIR or emits only a near-infrared light NIR. A wavelength of the red light may be in a range of 600 to 700 nm, and a wavelength of the near-infrared light may be in a range of 700 to 1,000 nm. Accordingly, the photodynamic therapy device according to the present invention may emit the red light and the near-infrared light in a wide wavelength range of 600 to 1,000 nm or may emit only the near-infrared light NIR in a range of 700 to 1,000 nm.

In the photodynamic therapy device including the tandem bio-OLED according to the present invention, the photodynamic therapy device includes a near-infrared pass dichroic filter (NPD) layer on the light conversion layer; or includes the NPD layer on the light conversion layer and a red-light-pass dichroic filter (RPD) layer below the light conversion layer. The NPD layer and the RPD layer may each be formed as a unit element in which a low refractive index layer and a high refractive index layer are repeated.

In the photodynamic therapy device including the tandem-structured bio-OLED according to the present invention, the light conversion layer includes an adhesive polymer layer and quantum dots (QD), a dye or a mixture of quantum dots and dye as a light conversion material formed on the adhesive polymer layer. The quantum dots may have a single structure or a core-shell dual structure. For example, the quantum dots having a single structure may include at least one of: MgO, MgS, MgSe, MgTe, CaO, CaS, CaSe, CaTe, SrO, SrS, SrSe, SrTe, BaO, BaS, BaSe, BaTE, ZnO, CuO, Cu₂O, ZnS, ZnSe, ZnTe, CdO, CdS, CdSe, CdTe, HgO, HgS, HgSe, HgTe, Al₂S₃, Al₂Se₃, Al₂Te₃, Ga₂O₃, Ga₂S₃, Ga₂Se₃, Ga₂Te₃, In₂O₃, In₂S₃, In₂Se₃, In₂Te₃, GeO₂, SnO₂, SnS, SnSe, SnTe, PbO, PbO₂, PbS, PbSe, PbTe, AlN, AlP, AlAs, AlSb, GaN, GaP, GaAs, GaSb, InN, InP, InAs and InSb. For example, the quantum dots having a core-shell dual structure may include at least one of: CdTe/CdSe, CdSe/ZnTe, CdTe/ZnS, CdSe/ZnS, CdTe/ZnSe, InP/ZnSe, InP/ZnS, InP/ZnTe, CdSe/ZnSe, InP/GaAs, InGaAs/GaAs, PbTe/PbS, CuInS₂/ZnS, Co/CdSe, Zn/ZnO and Ag/TiO₂. The dye may be based on, for example: rhodamine, coumarin, acridine, fluorescein, erythrosine, anthraquinone, arylmethane, AZO, diazonium, nitro, nitroso, phthalocyanine, quinone-imine, thiazole, safranin, xanthene, or a combination thereof, but the present invention is not limited thereto. A shape of the light conversion material may be a bead, a fiber, or a rod, but the present invention is not limited thereto. The adhesive polymer may be selected from: polymethyl methacrylate (PMMA), polystyrene (PS), polycarbonate (PC), polyethylene oxide (PEO), polyisoprene (PIP), polybutadiene (PB), polyvinyl alcohol (PVA), poly ethersulfone (PES), polyimide (PI), cellulose triacetate (CA), or a combination thereof, but the present invention is not limited thereto.

In the photodynamic therapy device including the tandem-structured bio-OLED according to the present invention, the first electrode layer may be a transparent electrode serving as an anode of the tandem-structured OLED, and, for example, the first electrode 220 may include or be made of ITO.

In the photodynamic therapy device including the tandem-structured bio-OLED according to the present invention, the second electrode layer may be a metal electrode serving as a cathode of the tandem-structured OLED, and, for example, the second electrode 230 may include or be formed of a metal thin film.

In the photodynamic therapy apparatus including the tandem-structured bio-OLED according to the present invention, the charge generation layer may include or be formed of aluminum (Al) having a thickness in a range of 1 to 2 nm.

In the photodynamic therapy apparatus including the tandem-structured bio-OLED according to the present invention, at least two or more OLED unit layers may be formed between the first electrode layer and the second electrode layer.

In the photodynamic therapy device including the tandem-structured bio-OLED according to the present invention, the OLED unit layer may emit a broadband emission spectrum with a full width at half maximum ("FWHM") of about 80 nm or more, preferably 90 nm or more, and more preferably 100 nm or more.

In the photodynamic therapy device including the tandem-structured bio-OLED according to the present invention, the OLED unit layer may include at least one of: a hole injection layer (HIL), a hole transport layer (HTL), an emission layer (EML), a hole block layer (HBL), an electron transport layer (ETL) and an electron injection layer (EIL). For example, the OLED unit layer may include a hole injection layer (HIL) formed on the first electrode layer, a hole transport layer (HTL) formed on the hole injection layer, an emission layer (EML) formed on the hole transport layer and emitting a red light (RED), a hole block layer (HBL) formed on the emission layer, an electron transport layer (ETL) formed on the hole block layer, and an electron injection layer (EIL) formed on the electron transport layer.

In the photodynamic therapy device including the tandem-structured bio-OLED according to the present invention, the photodynamic therapy device may further include a light efficiency enhancing layer (e.g., a capping layer (CPL)) on a side opposite to the OLED unit layer from among one side of the first electrode layer or the second electrode layer.

In the photodynamic therapy device including the tandem-structured bio-OLED according to the present invention, the photodynamic therapy device is used for treating at least or more medical conditions of: acne, psoriasis, eczema, cancer, pre-cancer, depression, bulimia, actinic keratosis, thyroid disorders, seasonal affective disorders, circadian rhythm maintenance disorders, neuropathy, wrinkles, cellulite, sleep disorders, tremors associated with Parkinson's disease, poor hair growth, poor fertility, obesity, wounds, poor circulation, irritable bowel syndrome, colic, inflammation, arthritis, Reynaud's syndrome, and infections.

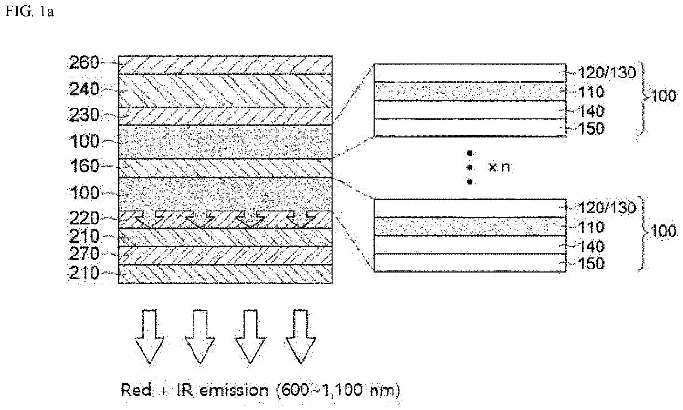

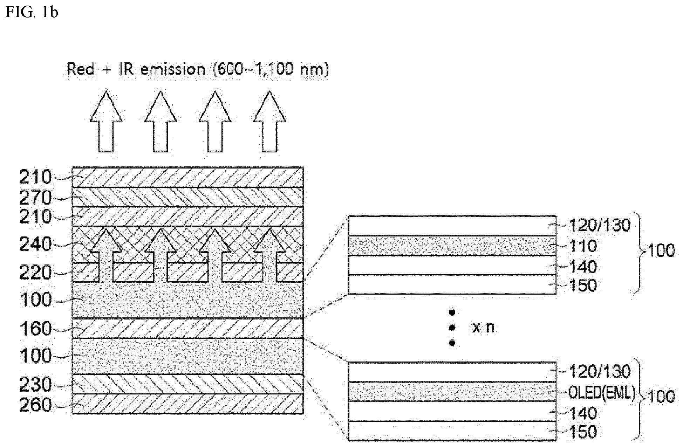

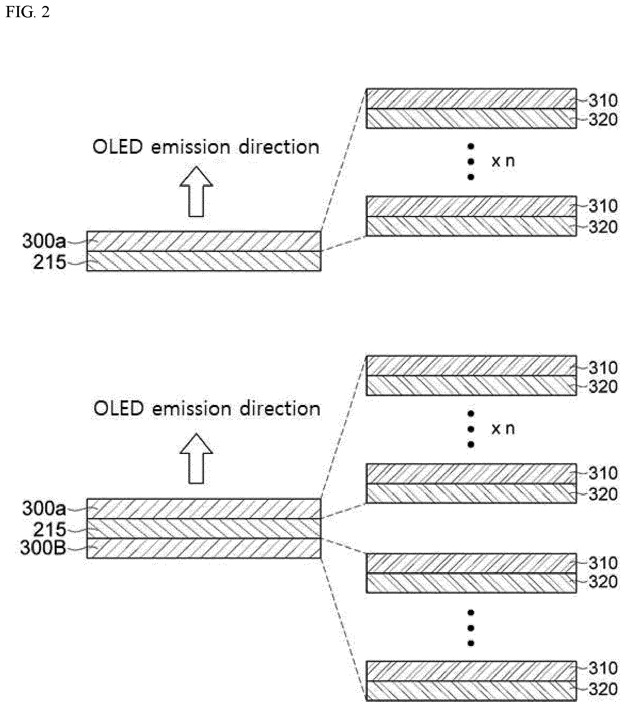

An exemplary embodiment of the photodynamic therapy device including the tandem-structured bio-OLED according to the present invention is illustrated in FIG. 1. For example, the photodynamic therapy device according to the present invention may include an encapsulation substrate layer 260; an OLED unit layer 100 including at least one of an EIL layer 120, an ETL layer 130, an EML layer 110, a HTL layer 140, and a HIL layer 150; and a light conversion layer 210. The bio-OLED according to the present invention may be classified into a bottom emission type and a top emission type according to positions of a first electrode layer 220 and a second electrode layer 230, and it is the bottom emission type (see FIG. 1a) when the first electrode layer 220 is disposed between the OLED unit layer 100 and a transparent encapsulation substrate layer 270 or a light conversion layer 210, and it is the top emission type (see FIG. 2b) when the transparent electrode layer 220 is disposed between the OLED unit layer 100 and a CPL 240 or a resonance layer.

Specifically, the photodynamic therapy device according to the present invention may be of a bottom emission type by being formed as: encapsulation substrate layer 260 / CPL layer 240 / second electrode layer 230 / OLED unit layer 100 / CGL layer 160 / OLED unit layer 100 / first electrode layer 220 / light conversion layer 210 / transparent encapsulation substrate layer 270 / light conversion layer 210, or may be of a top emission type by being formed as: encapsulation substrate layer 260 / second electrode layer 230 / OLED unit layer 100 / CGL layer 160 / OLED unit layer 100 / transparent electrode layer 220 / CPL layer 240 / light conversion layer 210 / transparent encapsulation substrate layer 270 / light conversion layer 210.

An exemplary embodiment of an NDP layer and an RDP layer of the bio-OLED for photodynamic therapy according to the present invention is illustrated in FIG. 2. According to the present invention, the red light emitted from the emission layer (EML) of the OLED is partially or entirely re-reflected from an electrode inside the OLED and from the NDP layer 300a, and accordingly, most of the red light may be photo-converted to a range of 700 nm to 1,000 nm by using a small amount of a light conversion material. In addition, a structure layer in which the red light is transmitted through the RPD layer 300b but the NIR light is reflected therefrom is provided such that a phenomenon in which the converted NIR light is trapped inside the OLED through scattering and reflection may be reduced, thereby increasing light output. The NPD layer and the RPD layer may be manufactured in a structure in which a unit including a low refractive index layer 310 and a high refractive index layer 320 is repeated into several layers with a thickness corresponding to a range of λ/7 to λ/2 with respect to the respective transmitted spectral regions. By using a relatively small amount of the material of the light conversion layer for the NIR light, the red light is reflected back inside the OLED from the NDP layer, and the photo-converted NIR light is transmitted through the NDP layer such that light conversion may be efficiently performed. Since a structure of the RPD layer 300a, the light conversion layer 215, and the NDP layer 300b is arranged in the order of the RPD layer 300a, the light conversion layer 215, and the NDP layer 300b on a side of an emission direction of the OLED, a red light transmitted from the light conversion layer is converted to a NIR light in the light conversion layer, and then, a reflected NIR light is not reflected back to the inside of the OLED, and is reflected from the RPD layer again to be guided to a corner of the OLED, thereby reducing degradation of the efficiency.

According to a third embodiment,
the present invention is directed to a tandem-structured bio-OLED array for photodynamic therapy, where the bio-OLED array includes:
a first electrode layer; a second electrode layer formed to face the first electrode; an OLED unit layer disposed between the first electrode layer and the second electrode layer and emitting a red light (RED); and a plurality of bio-OLEDs including a charge generation layer (CGL) disposed between each OLED unit layer,
where a light conversion layer including a light conversion material for converting the red light into a near-infrared light NIR emission is formed on a part of or all of the plurality of bio-OLEDs.

In the tandem-structured bio-OLED array for photodynamic therapy according to the present invention, at least two or more OLED unit layers may be formed between the first electrode layer and the second electrode layer.

In the tandem-structured bio-OLED array for photodynamic therapy according to the present invention, the OLED unit layer may emit a broadband emission spectrum with a full width at half maximum ("FWHM") of about 80 nm or more, preferably 90 nm or more, and more preferably 100 nm or more.

In the tandem-structured bio-OLED array for photodynamic therapy according to the present invention, the bio-OLED array may emit a red light and a near-infrared light NIR. A wavelength of the red light may be in a range of 600 to 700 nm, and a wavelength of the near-infrared light may be in a range of 700 to 1,000 nm. Accordingly, the bio-OLED array according to the present invention may emit the red light and the near-infrared light in a wide wavelength range of 600 to 1,000 nm.

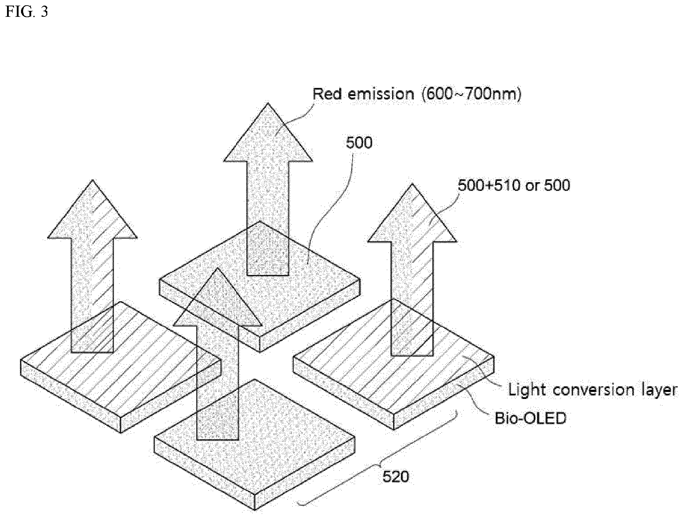

An exemplary embodiment of the tandem-structured bio-OLED array for photodynamic therapy according to the present invention is illustrated in FIG. 3. The bio-OLED array 520 may be formed as an array 520 of red OLEDs 500 emitting a light in a range of 600 nm to 700 nm and bio-OLEDs 500+510 including a light conversion layer for a photo-conversion to a range of 700 nm to 1,000 nm. Accordingly, the tandem-structured bio-OLED array for photodynamic therapy according to the present invention may enable the use of a PBM therapy in a wide wavelength range of 600 nm to 1,000 nm by including the red OLED and the bio-OLED including the light conversion layer which converts the red light into the near-infrared light NIR emission.

According to a fourth embodiment,
the present invention is directed to a method for manufacturing a light conversion film having excellent light conversion efficiency to near-infrared NIR, the method including:
(A) preparing spinning solutions by dissolving a light conversion material and an adhesive polymer in solvents, respectively; and
(B) spinning each of the spinning solutions by an electrospinning machine to which a voltage is supplied.

In the method for manufacturing the light conversion film having excellent light conversion efficiency according to the present invention, the light conversion material may include quantum dots (QD), a dye or a mixture of quantum dots and dye. The quantum dots may have a single structure or a core-shell dual structure. For example, the quantum dots having a single structure may include at least one of: MgO, MgS, MgSe, MgTe, CaO, CaS, CaSe, CaTe, SrO, SrS, SrSe, SrTe, BaO, BaS, BaSe, BaTE, ZnO, CuO, Cu2O, ZnS, ZnSe, ZnTe, CdO, CdS, CdSe, CdTe, HgO, HgS, HgSe, HgTe, Al₂S₃, Al₂Se₃, Al₂Te₃, Ga₂O₃, Ga₂S₃, Ga₂Se₃, Ga₂Te₃, In₂O₃, In₂S₃, In₂Se₃, In₂Te₃, GeO₂, SnO₂, SnS, SnSe, SnTe, PbO, PbO₂, PbS, PbSe, PbTe, AlN, AlP, AlAs, AlSb, GaN, GaP, GaAs, GaSb, InN, InP, InAs and InSb. For example, the quantum dots having a core-shell dual structure may include at least one of: CdTe/CdSe, CdSe/ZnTe, CdTe/ZnS, CdSe/ZnS, CdTe/ZnSe, InP/ZnSe, InP/ZnS, InP/ZnTe, CdSe/ZnSe, InP/GaAs, InGaAs/GaAs, PbTe/PbS, CuInS₂/ZnS, Co/CdSe, Zn/ZnO and Ag/TiO₂. The dye may be based on, but not limited to, for example: rhodamine, coumarin, acridine, fluorescein, erythrosine, anthraquinone, arylmethane, AZO, diazonium, nitro, nitroso, phthalocyanine, quinone-imine, thiazole, safranin, xanthene, or a combination thereof.

In the method for manufacturing the light conversion film having excellent light conversion efficiency according to the present invention, the adhesive polymer may be selected from: polymethyl methacrylate (PMMA), polystyrene (PS), polycarbonate (PC), polyethylene oxide (PEO), polyisoprene (PIP), polybutadiene (PB), polyvinyl alcohol (PVA), poly ethersulfone (PES), polyimide (PI), cellulose triacetate (CA), or a combination thereof, but the present invention is not limited thereto.

In the method for manufacturing the light conversion film having excellent light conversion efficiency according to the present invention, the solvent may be N-methyl-2-pyrrolidone (NMP), dimethylformamide (DMF), chloroform, dimethylsulfoxide, or N,N-dimethylacetamide (DMAc).

In the method for manufacturing the light conversion film having excellent light conversion efficiency according to the present invention, the voltage may be in a range of 9 to 30 kV.

In the method for manufacturing the light conversion film having excellent light conversion efficiency according to the present invention, a spinning rate may be in a range of 1.5 ml/h to 3 ml/h.

### MODES FOR IMPLEMENTATION OF THE INVENTION

### <Embodiment>

### Embodiment 1. Manufacturing of red OLED and property check

### 1-1. Manufacturing of red OLED

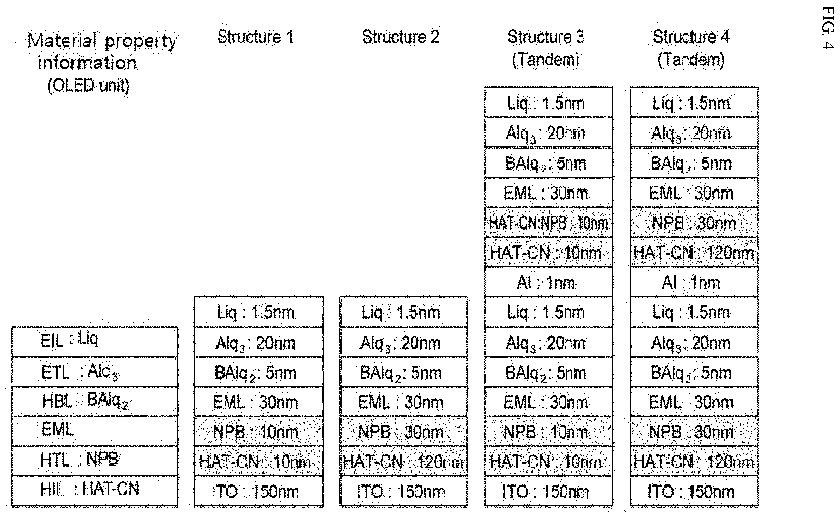

An ITO of 150 nm and 50×50 mm² was patterned as an anode electrode on a 50×50×1 mm³ glass substrate, four bottom-emission-type red OLED structures including a hole injection layer (HIL), a hole transport layer (HTL), an organic emission layer (EML), an electron transport layer (ETL), an electron injection layer (EIL), an electrode (cathode; Al), an encapsulation substrate (glass), and a hole block layer (HBL) were formed on the patterned anode ITO substrate, as illustrated in FIG. 4.

Structure 1: Liq(EIL) 1.5nm, Alq₃(ETL) 20nm, BAlq₂(HBL) 5nm, (EML)(Host:10%dopant) 25nm, NPB(HTL) 10nm, HAT-CN(HIL) 10nm

Structure 2: Liq(EIL) 1.5nm, Alq₃(ETL) 20nm, BAlq₂(HBL) 5nm, (EML)(Host:10%dopant) 25nm, NPB(HTL) 30nm, HAT-CN(HIL) 120nm

Structure3: Liq(EIL) 1.5nm, Alq₃(ETL) 20nm, BAlq₂(HBL) 5nm, (EML)(Host:10%dopant) 25nm, NPB(HTL) 10nm, HAT-CN((HIL), Anode) 10nm / Al(Cathode) 1nm, Liq(EIL) 1.5nm, Alq₃(ETL) 20nm, BAlq₂(HBL) 5nm, (EML)(Host:10%dopant) 25nm, NPB(HTL) 10nm, HAT-CN(HIL) 10nm

Structure 4: Liq(EIL) 1.5nm, Alq₃(ETL) 20nm, BAlq₂(HBL) 5nm, (EML)(Host:10%dopant) 25nm, NPB(HTL) 10nm, HAT-CN((HIL), Anode) 120nm / Al(Cathode)1nm, Liq(EIL) 1.5nm, Alq₃(ETL) 20nm, BAlq₂(HBL) 5nm, (EML)(Host: 10%dopant) 25nm, NPB(HTL) 30nm, HAT-CN(HIL) 120nm

### 1-2. Check voltage, luminance and optical power density of red OLED

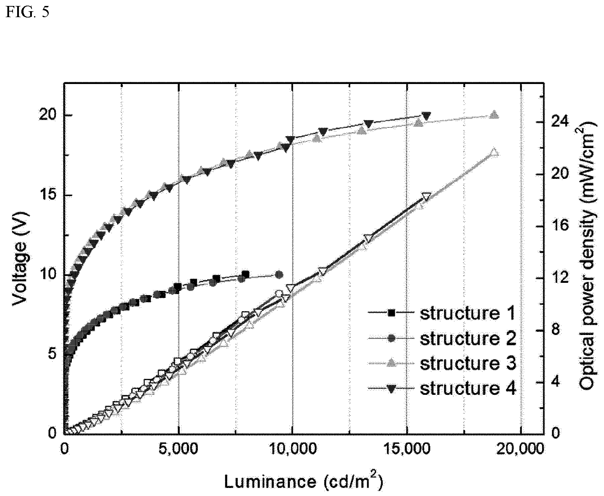

The voltage-luminance-optical power density of the four bottom-emission-type red OLED structures manufactured as above were checked and illustrated in FIG. 5. As a result, it was confirmed that the voltage, luminance, and optical power density of the red OLED structures 3 and 4 having a tandem structure was increased by about two times or more as compared to the red OLED structures 1 and 2 having a single stack.

### 1-3. Check spectral wavelength of red OLED

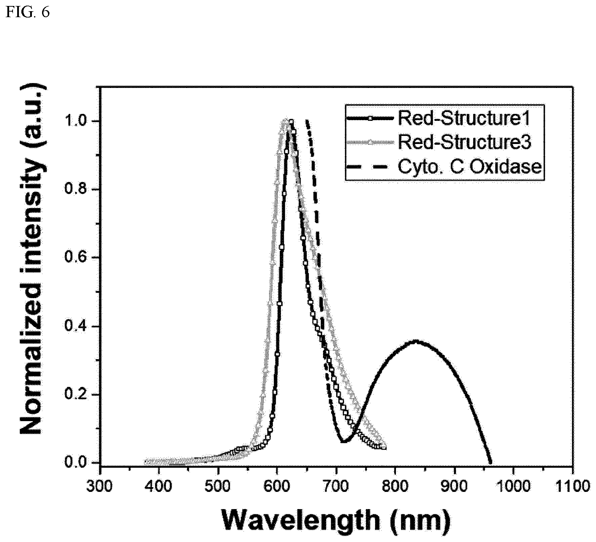

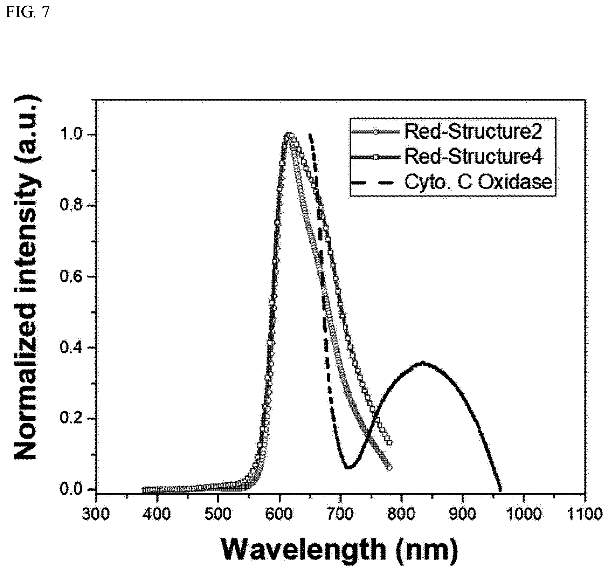

Spectral wavelengths of the bottom-emission-type red OLED structures 1 and 3 manufactured as above and spectral wavelengths of the red OLED structures 2 and 4 manufactured as above were checked, and illustrated in FIGS. 6 and 7, respectively. As a result, the red OLED structure 1 of a single stack had an overlap area of 16 % with cytochrome c oxidase, whereas, in the case of red OLED structure 3 having a tandem structure, an overlap area with cytochrome c oxidase increased to 20%. In addition, the red OLED structure 2 of a single stack had an overlap area with cytochrome c oxidase of 19%, whereas, in the case of the red OLED Structure 4 having a tandem structure, an overlap area with cytochrome c oxidase increased to 29%. Accordingly, it was confirmed that the OLED having a tandem structure according to the present invention has increased enzymatic compatibility as compared to the OLED of a single stack.

### Embodiment 2. Preparation of AgNW film

A glass and a PET film were thoroughly washed and dried over pure nitrogen. The glass substrate having a size of 5 cm × 5 cm was treated with octadecyltrichlorosilane dissolved in toluene for 24 hours to coat a self-assembled monolayer (SAM). An AgNW solution (0.5 wt%, IPA, Nanopyxis) was coated on the SAM-coated glass substrate by the Meyer rod method (rod No. 16) and then dried at 110°C. A polyacrylic acid (PAA) layer, which is a transparent adhesive component, was formed on a surface of a separately prepared PET film with a bar coater and cured at 60°C for 3 minutes. The AgNW formed on the hydrophobic SAM glass substrate was attached to a PAA PET substrate and transferred to a PET substrate to complete the AgNW film.

### Embodiment 3. Preparation of quantum dot beads and fibers

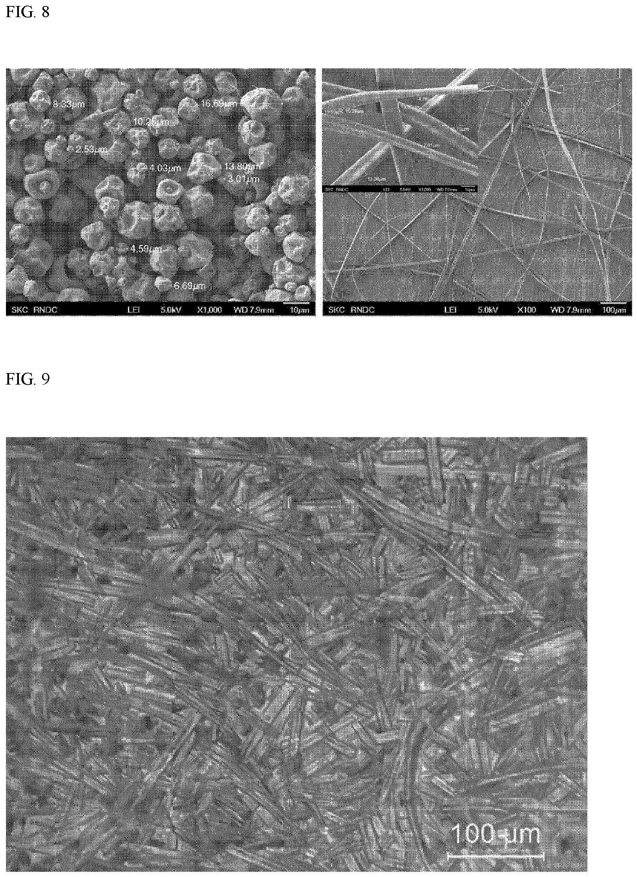

Quantum dot nanoparticles (CuInS₂ZnS (950 nm, FWHM 250 nm±30 nm, UBiQD) were dispersed in 10 mL of chloroform and 0.1 wt% of oleic acid using an ultrasonic vibrator as a solution. PMMA solutions in which PMMA and chloroform (PMMA:CHCl₃) were completely dissolved respectively at 1:9 and 2:8 were prepared and mixed with the dispersed CuInS₂ZnS solution at a ratio of 9:1 until they were completely dispersed to make two mixed solutions. The two solutions were respectively discharged at a rate of 2 ml/h, at a voltage of 20 kV, through nozzles having a distance of 10 cm with respect to the substrate to obtain beads and fibers, and they were observed by FE-SEM. As a result of spinning in the mixed solution of the solution having a PMMA and chloroform ratio of 1:9 and the CuInS₂ZnS solution, quantum dots in the form of beads having a size of about 3 µm to 17 µm were formed (FIG. 8a), thereby obtaining quantum dots. As a result of spinning in the mixed solution of the solution having a PMMA and chloroform ratio of 2:8 and the CuInS₂ZnS solution, quantum dots in the form of fibers (FIG. 8b) having a thickness of about 4 µm and a length of 1 mm or more were formed.

### Embodiment 4. Preparation of light conversion film

After coating about 4 µm of poly-acrylic-acid (PAA) through a bar coater on a 50 µm PET substrate, it was cured at a temperature of about 80°C for about 3 minutes. After spraying a quantum dot fiber with a thickness of about 4 µm on the PAA-coated PET substrate, a pressure of about 50 g/cm² was applied, a portion where the quantum dot fiber is not bonded was removed, and then the PAA was completely cured, thereby obtaining a single quantum dot fiber particle layer. Accordingly, as the PMMA component was broken and formed small in the process of bonding the quantum dot fiber with a length of 1 mm or more to the PET substrate through an adhesive transfer method, a light conversion film was formed in the form of a high-density rod bead (see FIG. 9).

### <Experimental example>

### Experimental Example 1. Analysis of spectral wavelength and light efficiency of red OLED with light conversion film attached

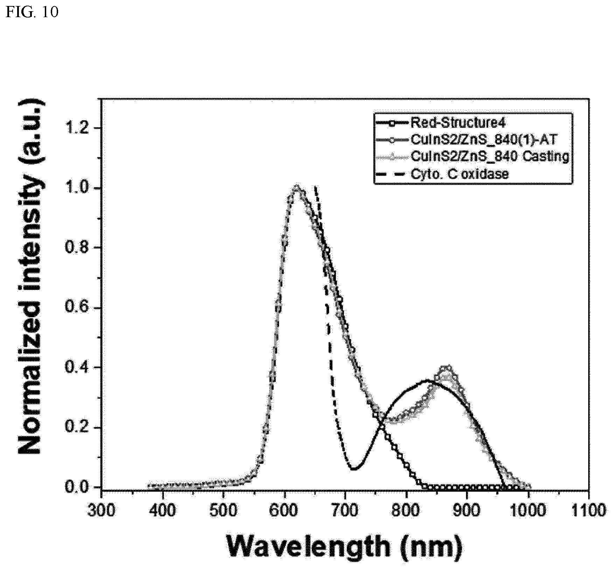

A spectral wavelength and a light efficiency of a light conversion module were analyzed by attaching light conversion films manufactured by an adhesive transfer (AT) process using CuInS₂/ZnS 950 nm to the red OLED structure 4 and a general casting process, respectively. As a result, CuInS₂/ZnS 950 nm emitted a light with respect to a center at 840 nm when excited by a red light (see FIG. 10). In addition, it was found that a light extraction efficiency (e.g., external quantum efficiency (EQE)) was increased by about 3% by attaching the light conversion film by the AT process on the red OLED. It was also confirmed that an increase in enzymatic compatibility in a range of about 13 % to 14 % was exhibited by the light conversion of the red OLED to which the light conversion film was attached (see Table 1).

**[Table 1]**

| | Red OLED | CIS(1)-AT | CIS Casting |
|---|---|---|---|
| conversion ratio | 0% | 22% | 21% |
| V | 13 | 13 | 13 |
| mA | 110 | 110 | 110 |
| EQE | 16% | 19% | 9% |
| Area(cm²) | 14.8 | 14.8 | 14.8 |
| mW/cm² | 15.5 | 18.2 | 8.5 |
| Overlap Area | 29% | 43% | 42% |
| EQE variation | 1.0 | 1.2 | 0.5 |

### Experimental Example 2. Analysis of spectral wavelength and light efficiency of red OLED with light conversion film attached

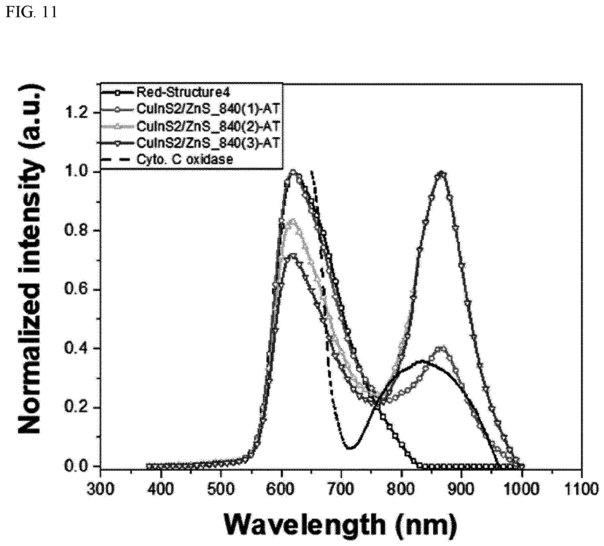

A spectral wavelength and a light efficiency of a light conversion module were analyzed by attaching light conversion films respectively prepared into one to three layers of CuInS₂/ZnS rod beads to the red OLED structure 4 by an adhesion transfer (AT) process (see FIG. 11). As a result, it was confirmed that when the light conversion film was attached, the enzymatic compatibility was 40 % or more, which was significantly increased as compared to the enzymatic compatibility of the red OLED structure of 29% (see Table 2).

**[Table 2]**

| | Red OLED | CIS(1) | CIS(2) | CIS(3) |
|---|---|---|---|---|
| conversion ratio | 0% | 16% | 34% | 46% |
| V | 13 | 13 | 13 | 13 |
| mA | 110 | 110 | 110 | 110 |
| EQE | 16% | 20% | 17% | 14% |
| Area(cm²) | 14.8 | 14.8 | 14.8 | 14.8 |
| mV/cm² | 15.5 | 19.5 | 16.4 | 13.8 |
| Overlap Area | 29% | 43% | 59% | 55% |
| EQE variation | 1.0 | 1.3 | 1.1 | 0.9 |

### Experimental Example 3. analysis of spectral wavelength and light efficiency of red OLED with light conversion film attached

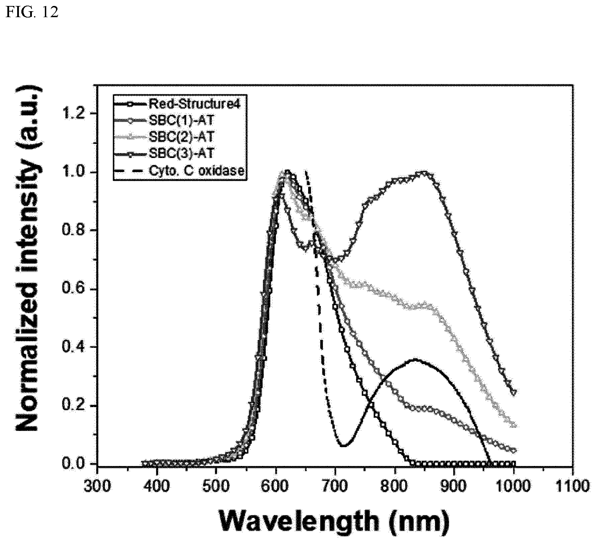

A spectral wavelength and a light efficiency of a light conversion module were analyzed by attaching light conversion films respectively prepared into one to three layers of ScBO₃:Cr³⁺ (SBC) fluorescent particle beads to the red OLED structure 4 by an adhesion transfer (AT) process (see FIG. 12). As a result, it was confirmed that when the light conversion film was attached, the enzymatic compatibility was 40 % or more, and particularly 85 % in the case of the light conversion film of three layers, which was significantly increased as compared to the enzymatic compatibility of the red OLED structure of 29 % (see Table 3).

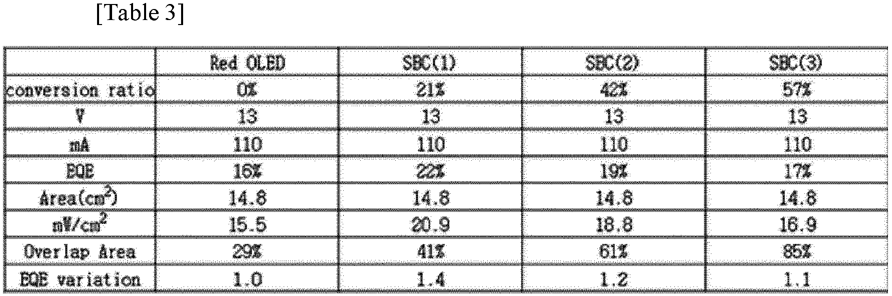

The present invention has been described with the embodiments. Those of ordinary skill in the art to which the present invention pertains will understand that the present invention may be implemented in a modified form without departing from the essential characteristics of the present invention. Therefore, the disclosed embodiments are to be considered in an illustrative rather than a restrictive sense. The scope of the present invention is indicated in the claims rather than the foregoing description, and all differences within the scope equivalent thereto should be construed as being included in the present invention.

## Claims

1. A tandem-structured bio-organic light emitting diode for photodynamic therapy comprising:
a first electrode layer;
a second electrode layer formed to face the first electrode layer;
an organic light emitting diode unit (OLED unit) layer disposed between the first electrode layer and the second electrode layer and emitting a red light (RED); and
a charge generation layer (CGL) disposed between each organic light emitting diode unit layer,
wherein at least two or more organic light emitting diode unit layers are formed between the first electrode layer and the second electrode layer.

2. The tandem-structured bio-organic light emitting diode for photodynamic therapy of claim 1, wherein the organic light emitting diode unit layer emits a broadband emission spectrum with a full width at half maximum of about 80 nm or more.

3. The tandem-structured bio-organic light emitting diode for photodynamic therapy of claim 1, wherein the organic light emitting diode unit layer comprises: a hole injection layer (HIL) formed on the first electrode layer, a hole transport layer (HTL) formed on the hole injection layer, an emission layer (EML) formed on the hole transport layer and emitting a red light (RED), a hole block layer (HBL) formed on the emission layer, an electron transport layer (ETL) formed on the hole block layer, and an electron injection layer (EIL) formed on the electron transport layer, and
the hole injection layer is formed to a thickness in a range of 1 to 150 nm, and the hole transport layer is formed to a thickness in a range of 1 to 50 nm.

4. A photodynamic therapy device comprising a tandem-structured bio-organic light emitting diode, comprising:
(A) an encapsulation substrate layer,
(B) a tandem-structured bio-organic light emitting diode (bio-OLED) layer comprising a first electrode layer on the encapsulation substrate layer; a second electrode layer formed to face the first electrode layer; at least two or more organic light emitting diode unit (OLED unit) layers disposed between the first electrode layer and the second electrode layer and emitting a red light (RED); and a charge generation layer (CGL) disposed between each organic light emitting diode unit layer,
(C) a transparent encapsulation substrate layer formed on the bio-organic light emitting diode layer, and
(D) a light conversion layer on, or on and below the transparent encapsulation substrate layer,
wherein the light conversion layer converts the red light emitted from the bio-organic light emitting diode layer into a near-infrared (NIR) emission.

5. The photodynamic therapy device comprising the tandem-structured bio-organic light emitting diode of claim 4, wherein the photodynamic therapy device emits a red light and a near-infrared light (NIR) or emits only a near-infrared light (NIR).

6. The photodynamic therapy device comprising the tandem-structured bio-organic light emitting diode of claim 4, wherein the photodynamic therapy device comprises a near-infrared-light-pass dichroic filter (NIR-pass-dichroic filter, NPD) layer on the light conversion layer; or comprises the NPD layer on the light conversion layer and a red-light-pass dichroic filter (RPD) layer below the light conversion layer.

7. The photodynamic therapy device comprising the tandem-structured bio-organic light emitting diode of claim 6, wherein the NPD layer and the RPD layer are each formed as a unit in which a low refractive index layer and a high refractive index layer are repeated.

8. The photodynamic therapy device comprising the tandem-structured bio-organic light emitting diode of claim 4, wherein the light conversion layer comprises an adhesive polymer layer; and quantum dots (QD), a dye or a mixture of quantum dots and dye as a light conversion material formed on the adhesive polymer layer.

9. A tandem-structured bio-organic light emitting diode array for photodynamic therapy, comprising:
a plurality of bio-organic light emitting diodes (bio-OLEDs) comprising: a first electrode layer; a second electrode layer formed to face the first electrode layer; at least two or more organic light emitting diode unit (OLED unit) layers disposed between the first electrode layer and the second electrode layer and emitting a red light (RED); and a charge generation layer (CGL) disposed between each organic light emitting diode unit layer,
wherein a light conversion layer comprising a light conversion material for converting the red light into a near-infrared light (NIR) is formed on a part of or all of the plurality of bio-organic light emitting diodes.

10. The tandem-structured bio-organic light emitting diode array of claim 9, emitting a red light and a near-infrared light in a wide wavelength range of 600 to 1,000 nm.
